# EUROPÄISCHE PATENTSCHRIFT

(11) **EP 0 324 022 B1**
(45) Veröffentlichungstag und Bekanntmachung des Hinweises auf die Patenterteilung: **15.09.1993**
(21) Anmeldenummer: 88907052.0
(22) Anmeldetag: 08.07.1988
(51) Int. Cl.: A61B 17/56, A61F 2/44

(54) **POSITIONIERUNGSVORRICHTUNG**
POSITIONING DEVICE
DISPOSITIF DE POSITIONNEMENT

(30) Priorität: 08.07.1987 DE 3722590; 04.01.1988 DE 3800052
(43) Veröffentlichungstag der Anmeldung: 19.07.1989
(73) Patentinhaber: Biedermann, Lutz, D-78054 Villingen-Schwenningen (DE); Harms, Jürgen, Prof. Dr., 76337 Waldbronn (DE)
(72) Erfinder: Biedermann, Lutz, D-78054 Villingen-Schwenningen (DE); Harms, Jürgen, Prof. Dr., 76337 Waldbronn (DE)
(74) Vertreter: Prüfer, Lutz H., Dipl.-Phys.
(86) Internationale Anmeldenummer: EP8800617
(87) Internationale Veröffentlichungsnummer: WO8900028

(56) Entgegenhaltungen:
- DE-B- 2 649 042
- DE-U- 8 610 858

## Beschreibung

Die Erfindung betrifft eine Positionierungsvorrichtung zum Stabilisieren von Wirbelsäulensegmenten gemäß dem Oberbegriff des Anspruchs 1 bzw. des Anspruchs 6. Derartige Vorrichtungen werden zur Stabilisierung der Wirbelsäule oder auch zur Stabilisierung eines Platzhalters zwischen zwei benachbarten Wirbelkörpern verwendet.

Eine Positionierungsschraube ist aus der DE-AS 26 49 042 bekannt. Die Schraube weist einen Gewindeteil und einen starr damit am kopfseitigen Ende vorgesehenen Aufnahmeteil auf. Die jeweiligen Aufnahmeteile weisen Aufnahmeschlitze auf. Die seitlichen Begrenzungen der Schlitze werden durch ebene Flächen mit konzentrischen Vertiefungen gebildet. Die angreifenden Muttern weisen ebene Anlageflächen mit konzentrischen Kragen auf. Die Kragen greifen in die Vertiefungen und die ebenen Flächen der Muttern wirken mit den ebenen Anlageflächen des Aufnahmeteiles zusammen. Dadurch wird verhindert, daß die Stange aus dem Aufnahmeteil herausgelangt. Ferner wird die Aufnahmestange in einer Position senkrecht zur Achse des Aufnahmeteiles fixiert. Es sind den Schraubenköpfen unterlegbare und der Form der Wirbelkörper etwa angepaßte Druckverteilungsplatten vorgesehen. Ein Nachteil dieser Lösung besteht darin, daß es sehr schwierig ist, die Schrauben einerseits fest in die Wirbelkörper einzuschrauben und andererseits die Schrauben in zwei Ebenen gerade so zu stellen, daß die Achsen der Aufnahmeschlitze in den übereinander befindlichen Aufnahmeteilen so ausgerichtet sind, daß die Gewindestange ohne Verspannung der Schrauben durch die Aufnahmeschlitze hindurchführbar ist. Schon der Versuch erfordert sehr viel Zeit, was bei einer Operation an der Wirbelsäule ein großer Nachteil ist. Darüber hinaus läßt sich eine so genaue Ausrichtung fast nicht erreichen. Das Ergebnis ist, daß erhebliche Scherkräfte auf die Gewindestangen ausgeübt werden, was dazu führt, daß in der späteren Benutzung nach Abschluß der Operation die Stangen sogar abbrechen können bzw. nicht die volle Stabilisierung ermöglichen. Die erforderlichen Druckverteilungsplatten sind darüber hinaus zusätzliche bewegliche Teile, deren Anbringung und Handhabung die Operation erschweren.

Aufgabe der Erfindung ist es, eine Positionierungsvorrichtung der eingangs beschriebenen Art zu schaffen, die das Einsetzen erleichtert und gleichzeitig auch die Gefahren beim späteren Benutzen vermindert bzw. ausschließt.

Diese Aufgabe wird durch eine Positionierungsvorrichtung der eingangs beschriebenen Art gelöst, die durch die Merkmale der kennzeichnenden Teile der Patentansprüche 1 bzw. 6 gelöst werden.

Weiterbildungen der Erfindung ergeben sich aus der Beschreibung von Ausführungsbeispielen anhand der Figuren. Von den Figuren zeigen:
- Fig. 1: eine Seitenansicht der Positionierungsvorrichtung senkrecht zur Längsachse der Stange, ohne rechtes Halteelement;
- Fig. 2: eine Seitenansicht senkrecht zu der in Figur 1 gezeigten Darstellung, bei der die Halteelemente und das Aufnahmeteil geschnitten sind;
- Fig. 3: eine der Fig. 1 entsprechende Darstellung einer zweiten Ausführungsform;
- Fig. 4: eine der Fig. 2 entsprechende Darstellung der zweiten Ausfuhrungsform;
- Fig. 5: eine Seitenansicht einer dritten Ausführungsform; und
- Fig. 6: eine der Fig. 1 entsprechende Darstellung der dritten Ausführungsform.

Die Positionierungsvorrichtung umfaßt eine Positionierungsschraube 1 mit einem Gewindeschaftteil 2 und einem Aufnahmeteil 3.

Das Aufnahmeteil 3 umfaßt einen kugelförmigen Kopf. Dieser weist einen sich im wesentlichen senkrecht zur Achse des Gewindeschaftteiles 2 erstreckenden Schlitz 4 auf. Der Schlitz nimmt eine Stange 5 auf. Die Stange 5 ist als Gewindestange ausgebildet. Zur Fixierung der Stange in dem Schlitz sind zwei Muttern 6, 7 vorgesehen, die auf dem Stange 5 geführt von außen an dem Kopf angreifen.

Die Mittenebene des Schlitzes fällt mit dem Mittelpunkt des kugelförmigen Kopfes zusammen. Der Schlitz erstreckt sich um mehr als einen halben Durchmesser der Stange über den Mittelpunkt des kugelförmigen Kopfes hinaus in den Kopf hinein, so daß es möglich ist, daß die Stange mit ihrer Mittenachse um den Kugelmittelpunkt in einem gewünschten Winkelbereich in Richtung der Mittenebene des Schlitzes schwenkbar ist.

Die Breite des Schlitzes 4 ist größer als der Durchmesser der Stange 5, so daß es möglich ist, die Stange mit ihrer Mittenachse um den Mittelpunkt des kugelförmigen Kopfes auch in einer Richtung senkrecht zu der erstgenannten Schwenkrichtung innerhalb eines vorbestimmten Winkelbereiches zu schwenken.

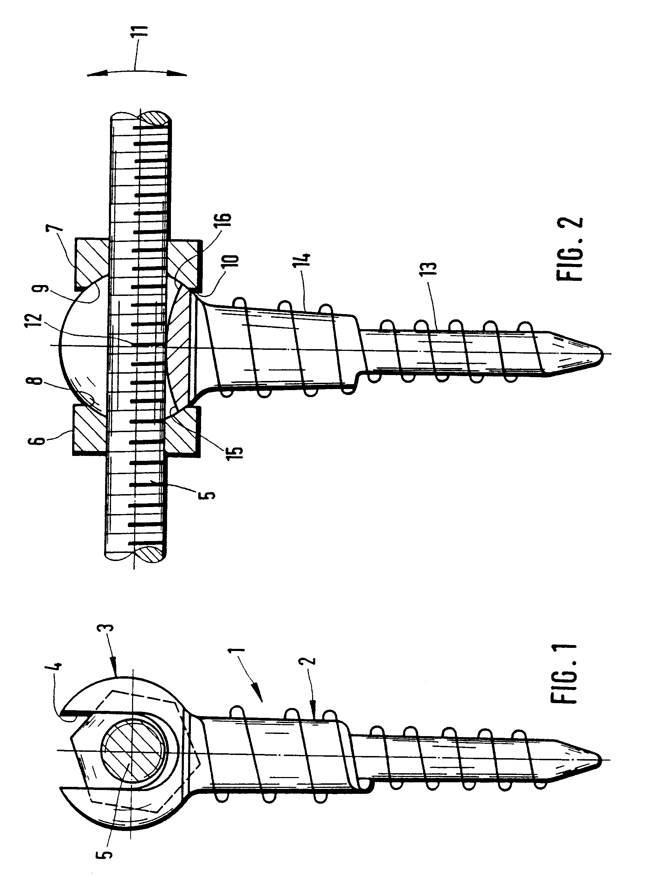

Wie am besten aus Figur 2 ersichtlich ist, weisen bei der ersten Ausführungsform die Muttern 6, 7 auf ihren dem kugelförmigen Kopf zugewandten Seiten Widerlagerflächen in Form hohlkugelsegmentförmiger Flächen 8, 9 auf. Der Radius dieser Flächen ist im wesentlichen gleich dem Radius des kugelförmigen Kopfes gebildet, so daß die Muttern vollflächig an der Wandung des kugelförmigen Bereiches anliegen.

Wie am besten aus Figur 2 ersichtlich ist, sind die Muttern so ausgebildet, daß ihr unterer Rand 10 einen Abstand von dem daran angrenzenden Bereich des Gewindeschaftteiles 2 aufweist, so daß ein Verschwenken der Stange 5 in Richtung des Pfeiles 11 um den Kugelmittelpunkt 12 herum nicht behindert wird.

Der Gewindeschaftteil 2 weist an seinem dem Aufnahmeteil 3 gegenüberliegenden Ende einen Abschnitt 13 mit einem ersten Durchmesser auf. Dieser erstreckt sich über den größten Teil der Länge des Gewindeschaftteiles. Zwischen diesem Abschnitt und dem Aufnahmeteil 3 erstreckt sich ein weiterer ein Gewinde aufweisender Abschnitt mit einem größeren Durchmesser bzw. größeren Gewinde.

Im Betrieb wird zunächst die Positionierungsschraube in den Wirbelköper oder einen Platzhalter zwischen zwei Wirbelkörpern eingeschraubt. Durch das Vorsehen der zwei Abschnitte 13 und 14 mit verschieden starken Gewinden wird die Positionierungsschraube so fest eingeschraubt, daß es nicht mehr erforderlich ist, zusätzliche Unterlegscheiben vorzusehen. Anschließend wird die Stange 5 in den Schlitz der Positionierungsschraube und gleichzeitig in die Schlitze der übrigen die Stange führenden Positionierungsschrauben eingelegt. Aufgrund der beschriebenen Ausführung der Schlitze ist eine Ausrichtung der Stange auch abweichend von einer Richtung senkrecht zur Längsachse des Gewindeschaftteiles möglich. Anschließend werden die Muttern 6, 7 so angezogen, daß sie an dem Aufnahmeteil in der in Figur 2 gezeigten Weise anliegen. Die Muttern werden dann so fest angezogen, daß eine Art Kaltverschweißung mit den damit zusammenwirkenden Bereichen des Aufnahmeteiles gebildet wird.

In der obigen Beschreibung hat der Schlitz einen geraden Boden. Bevorzugt ist der Schlitzboden aber in der aus den Figuren ersichtlichen Weise von der Schlitzmitte aus in Längsrichtung des Schlitzes zu den beiden äußeren Enden 15, 16 hin abgesenkt. Vorzugsweise weist der Boden in der mit der Schlitzachse zusammenfallenden Ebene im wesentlichen einen Radius um einen Mittelpunkt auf, der auf der den Mittelpunkt 12 des Kugelsegmentes gegenüberliegenden Seite liegt. Dadurch wird erreicht, daß die Stange 5 um den Mittelpunkt 12 des Kugelsegmentes um einen relativ großen Winkel schwenkbar ist.

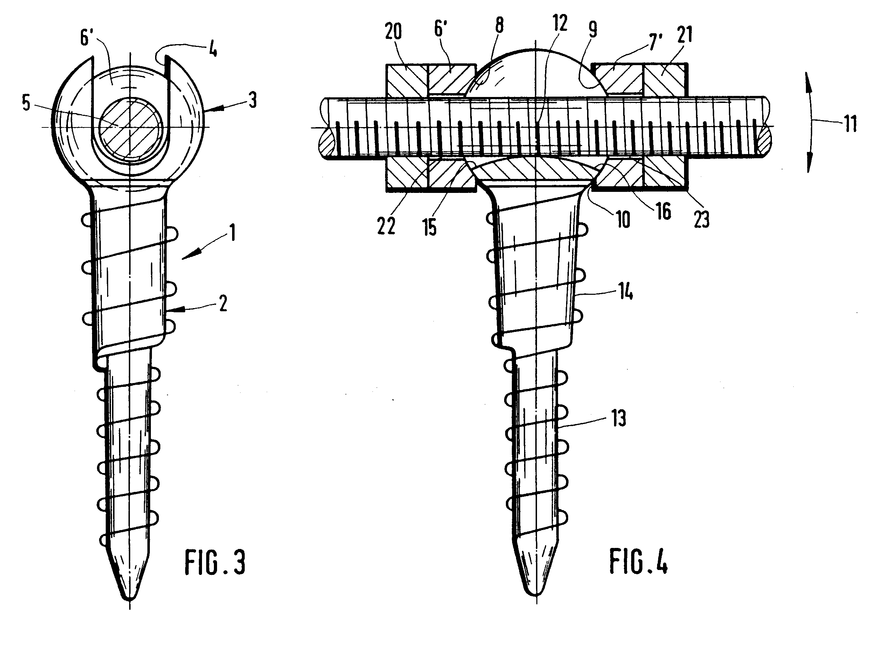

Bei der oben beschriebenen ersten Ausführungsform greifen die Muttern direkt an das Aufnahmeteil an. Bei der in den Figuren 3 und 4 gezeigten Ausführungsform sind die an dem Aufnahmeteil 3 anliegenden Halteelemente als auf der Stange 5 hin- und herbewegbare scheibenförmige Zwischenstücke aus 6', 7' ausgebildet, denen auf der jeweiligen dem Aufnahmeteil 3 abgewandten Seite Muttern 20, 21 angreifen. Die Zwischenstücke 6', 7' haben auf ihrer dem Aufnahmeteil 3 zugewandten Seite jeweils die gleiche Form wie die oben beschriebenen Muttern. Anstelle des Innengewindes ist jeweils eine Bohrung 22, 23 vorgesehen, deren Durchmesser so groß ist, daß ein Gleiten auf der Stange 5 möglich ist.

Die mit dem Aufnahmeteil 3 zusammenwirkenden Innenflächen der Zwischenstücke 6', 7' sind vorzugsweise derart aufgerauht, daß bei Anziehen der Muttern 20, 21 die Arretierungsstellung ein Kaltverschweißen zwischen den Zwischenstücken und dem Aufnahmeteil 3 eine feste Verbindung ergibt.

Im Betrieb wird in gleicher Weise vorgegangen wie bei der ersten Ausführungsform.

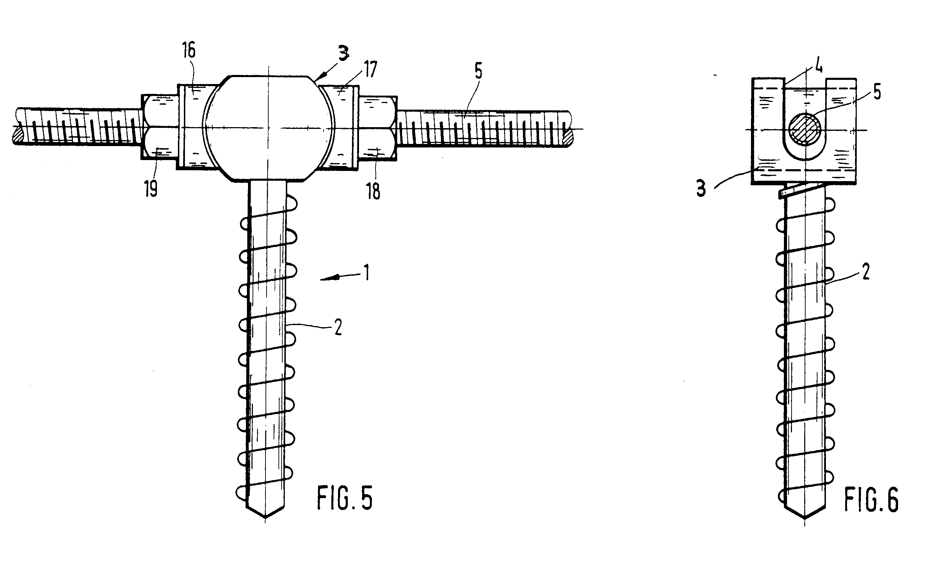

Bei der in den Figuren 5 und 6 gezeigten Ausführungsform weist das Aufnahmeteil 3 einen zylinderförmigen Kopf auf. Dieser weist einen sich im wesentlichen parallel zur Achse des Gewindeschaftteiles 2 erstreckenden Schlitz 4 auf. Der Schlitz nimmt wiederum die Stange 5 auf, die als Gewindestange ausgebildet ist. Zur Fixierung der Stange sind zwei Zwischenglieder 16, 17 und an diesem angreifende Muttern 18, 19 vorgesehen, die auf der Stange 5 geführt von außen an dem Kopf angreifen.

Der Schlitz 4 erstreckt sich in einer Ebene, die im wesentlichen senkrecht zur Zylinderachse des zylinderförmigen Kopfes liegt. Ansonsten stimmt die Ausbildung des Schlitzes und seine Anordnung mit der oben beschriebenen Ausführungsform überein.

Die Zwischenglieder 16, 17 weisen auf ihren dem zylinderförmigen Kopf zugewandten Seiten Widerlagerflächen in Form von hohlzylinderförmigen Segmenten auf, deren Krümmungsradius im wesentlichen dem Krümmungsradius der angrenzenden Zylindersegmentflächen des Aufnanmeteiles entspricht, so daß die Zwischenglieder an der Wandung der jeweiligen zylindersegmentförmigen Abschnitte des Aufnahmeteiles 3 anliegen. Die Zwischenglieder 16, 17 weisen im wesentlichen durch den Mittelpunkt der Zylindersegmentflächen und senkrecht zur Zylinderachse sich erstreckende Bohrungen auf, deren Durchmesser so gewählt ist, daß die Zwischenglieder frei auf der Stange 5 hin- und herbewegbar sind.

Wie am besten aus Figur 5 ersichtlich ist, greifen die Muttern 18, 19 auf den dem Aufnahmeteil 3 abgewandten Außenflächen der Zwischenglieder 16, 17 so an, daß über die Muttern 18, 19 und die Zwischenglieder 16, 17 eine feste Verbindung zwischen der Stange 5 und der Positionierungsschraube 1 hergestellt wird.

Im Betrieb wird in gleicher Weise vorgegangen, wie bei der ersten Ausführungsform.

## Patentansprüche

1. Positionierungsvorrichtung zum Stabilisieren von Wirbelsäulensegmenten,
mit einem Gewindeschaftteil (2) und einem kopfseitig vorgesehenen Aufnahmeteil (3) für eine Stange (5), die in einen im Aufnahmeteil (3) vorgesehenen Schlitz (4) eingreift, und wobei die Stange (5) und das Aufnahmeteil (3) durch zwei an dem Aufnahmeteil angreifende Halteelemente (6, 7) fest miteinander verbindbar sind,
dadurch gekennzeichnet, daß der jeweilige das Widerlager für die Halteelemente (6, 7; 6', 7'; 20, 21) bildende Teil des Aufnahmeteiles (3) als Kugelsegment ausgebildet ist, und die damit in Eingriff bringbare Fläche (8, 9) wenigstens eines der Halteelemente (6, 7; 6', 7'; 20, 21) eine zum Kugelsegment des Halteelementes konzentrische hohlkugelförmige Oberfläche aufweist.

2. Positionierungsvorrichtung nach Anspruch 1,
dadurch gekennzeichnet, daß beide Halteelemente (6, 7; 6', 7') eine entsprechende hohlkugelförmige Oberfläche aufweisen.

3. Positionierungsvorrichtung nach Anspruch 2,
dadurch gekennzeichnet, daß der Radius der hohlkugelförmigen Oberfläche im wesentlichen gleich dem Radius des zugehörigen Kugelsegmentes ist.

4. Positionierungsvorrichtung nach Anspruch 1,
dadurch gekennzeichnet, daß die Halteelemente (6, 7) als Zwischenscheiben (6', 7') ausgebildet sind.

5. Positionierungsvorrichtung nach Anspruch 1,
dadurch gekennzeichnet, daß die Halteelemente (6, 7) als Muttern ausgebildet sind.

6. Positionierungsvorrichtung zum Stabilisieren von Wirbelsäulensegmenten,
mit einem Gewindeschaftteil (2) und einem kopfseitig vorgesehenen Aufnahmeteil (3) für eine Stange (5), die in einen im Aufnahmeteil (3) vorgesehenen Schlitz (4) eingreift, und wobei die Stange (5) und das Aufnahmeteil (3) durch zwei Muttern (18, 19) fest miteinander verbindbar sind,
dadurch gekennzeichnet, daß der Aufnahmeteil (3) zylindersegmentförmig ausgebildet ist und zwischen Aufnahmeteil (3) und Muttern (18, 19) jeweils ein Zwischenglied (16, 17) mit einer dem Aufnahmeteil (3) zugewandten und der Zylinderform im wesentlichen entsprechenden hohlzylinderförmigen Oberfläche vorgesehen ist.

7. Positionierungsvorrichtung nach einem der Ansprüche 1 oder 6,
dadurch gekennzeichnet, daß die Mittenebene des Schlitzes (4) durch den Mittelpunkt (12) des Kugelsegmentes bzw. die Längsachse des Zylindersegmentes geht.

8. Positionierungsvorrichtung nach Anspruch 1 oder 6,
dadurch gekennzeichnet, daß der Schlitz (4) sich um mehr als einen halben Durchmesser der Stange über den Mittelpunkt (12) des Aufnahmeteiles (3) in dieses hinein erstreckt.

9. Positionierungsvorrichtung nach Anspruch 1 oder 6,
dadurch gekennzeichnet, daß die Breite des Schlitzes (4) größer als der Durchmesser der Stange (5) ist.

10. Positionierungsvorrichtung nach Anspruch 1 oder 6,
dadurch gekennzeichnet, daß der Gewindeschaft (2) an seinem dem Aufnahmeteil (3) abgewandten Ende einen Abschnitt (13) mit einem ersten Durchmesser und daran angrenzend einen Abschnitt (14) mit einem zweiten Durchmesser, der größer ist als der erste Durchmesser, aufweist.

11. Positionierungsvorrichtung nach Anspruch 1 oder 6,
dadurch gekennzeichnet, daß die Ebene des Schlitzes (4) sich im wesentlichen parallel zu der Längsachse des Gewindeschaftteiles (2) erstreckt.

12. Positionierungsvorrichtung nach Anspruch 1 oder 6,
dadurch gekennzeichnet, daß die äußeren Seiten des Bodens des Schlitzes (4) so abgesenkt sind, daß eine Verschwenkung der Stange (5) um den Mittelpunkt (12) des Kugelsegmentes bzw. um die Achse des Zylindersegmentes möglich ist.

## Claims

1. Positioning device for stabilising vertebral column segments, having a threaded shank part (2) and a receiving part (3), provided at the head end, for a rod (5) which engages into a slot (4) provided in the receiving part (3), and the rod (5) and the receiving part (3) being able to be firmly connected to each other by two holding elements (6, 7) acting upon the receiving part, characterised in that the respective part of the receiving part (3) which forms the abutment for the holding elements (6, 7; 6', 7'; 20, 21) is configured as a spherical segment and in that the surface area (8, 9), which can be brought into engagement therewith, of at least one of the holding elements (6, 7; 6', 7'; 20, 21) exhibits a hollow-sphere-shaped surface concentric to the spherical segment of the holding element.

2. Positioning device according to Claim 1, characterised in that both holding elements (6, 7; 6', 7') exhibit a corresponding hollow-sphere-shaped surface.

3. Positioning device according to Claim 2, characterised in that the radius of the hollow-sphere-shaped surface is essentially equal to the radius of the associated spherical segment.

4. Positioning device according to Claim 1, characterised in that the holding elements (6, 7) are configured as shims (6', 7').

5. Positioning device according to Claim 1, characterised in that the holding elements (6, 7) are configured as nuts.

6. Positioning device for stabilising vertebral column segments, having a threaded shank part (2) and a receiving part (3), provided at the head end, for a rod (5) which engages into a slot (4) provided in the receiving part (3), and the rod (5) and the receiving part (3) being able to be firmly connected to each other by two nuts (18, 19), characterised in that the receiving part (3) is configured in the shape of a cylindrical segment and in that between the receiving part (3) and the nuts (18, 19) there is provided in each case an intermediate member (16, 17) having a hollow-cylinder-shaped surface facing the receiving part (3) and essentially corresponding to the cylinder shape.

7. Positioning device according to one of Claims 1 or 6, characterised in that the centre plane of the slot (4) passes through the midpoint (12) of the spherical segment or the longitudinal axis of the cylindrical segment.

8. Positioning device according to Claim 1 or 6, characterised in that the slot (4) extends by more than half the diameter of the rod over the midpoint (12) of the receiving part (3) and into the latter.

9. Positioning device according to Claim 1 or 6, characterised in that the width of the slot (4) is greater than the diameter of the rod (5).

10. Positioning device according to Claim 1 or 6, characterised in that the threaded shank (2) exhibits, at its end facing away from the receiving part (3), a section (13) having a first diameter and, adjacent thereto, a section (14) having a second diameter which is greater than the first diameter.

11. Positioning device according to Claim 1 or 6, characterised in that the plane of the slot (4) extends essentially parallel to the longitudinal axis of the threaded shank part (2).

12. Positioning device according to Claim 1 or 6, characterised in that the outer sides of the base of the slot (4) are depressed such that the rod (5) can be pivoted about the midpoint (12) of the spherical segment or about the axis of the cylindrical segment.

## Revendications

1. Dispositif de positionnement pour la stabilisation de segments du rachis, comportant une partie formant corps fileté (2) et une partie de réception (3) prévue du côté de la tête pour une tige (5) qui se met en prise dans une fente (4) prévue dans la partie de réception (3), et dans lequel la tige (5) et la partie de réception (3) peuvent être reliées solidement par des éléments de maintien (6, 7) se mettant en prise sur la partie de réception, caractérisé en ce que la zone de la partie de réception (3) formant butée pour chacun des éléments de maintien (6, 7 ; 6', 7' ; 20, 21) est conçue comme un segment de sphère, et en ce que la surface (8, 9) d'au moins l'un des éléments de maintien (6, 7 ; 6', 7' ; 20, 21) pouvant être mise en prise avec ladite zone formant butée présente une surface en forme de sphère creuse concentrique au segment de sphère de l'élément de maintien.

2. Dispositif de positionnement selon la revendication 1, caractérisé en ce que les deux éléments de maintien (6, 7 ; 6', 7') présentent une surface en forme de sphère creuse correspondante.

3. Dispositif de positionnement selon la revendication 2, caractérisé en ce que le rayon de la surface en forme de sphère creuse est sensiblement égal au rayon de segment de sphère correspondant.

4. Dispositif de positionnement selon la revendication 1, caractérisé en ce que les éléments de maintien (6, 7) sont conçus comme des disques intercalaires (6', 7').

5. Dispositif de positionnement selon la revendication 1, caractérisé en ce que les éléments de maintien (6, 7) sont conçus comme des écrous.

6. Dispositif de positionnement pour la stabilisation de segments du rachis, comportant une partie formant corps fileté (2) et une partie de réception (3) prévue du côté de la tête pour une tige (5) qui se met en prise dans une fente (4) prévue dans la partie de réception (3), et dans lequel la tige (5) et la partie de réception (3) peuvent être reliées solidement par deux écrous (18, 19), caractérisé en ce que la partie de réception (3) est de forme cylindrique et en ce qu'il est prévu entre la partie de réception (3) et les écrous (18, 19) des éléments intermédiaires (16, 17) pourvus d'une surface en forme de cylindre creux orientée vers la partie de réception (3) et correspondant sensiblement à la forme du cylindre.

7. Dispositif de positionnement selon l'une quelconque des revendications 1 ou 6, caractérisé en ce que le plan médian de la fente (4) passe par le centre (12) du segment de sphère ou l'axe longitudinal du segment de cylindre.

8. Dispositif de positionnemet selon l'une quelconque des revendications 1 ou 6, caractérisé en ce que la fente (4) s'étend sur plus d'un demi-diamètre de la tige au-delà du centre (12) de la partie de réception (3) vers l'intérieur de celle-ci.

9. Dispositif de positionnement selon l'une quelconque des revendications 1 ou 6, caractérisé en ce que la largeur de la fente (4) est plus grande que le diamètre de la tige (5).

10. Dispositif de positionnement selon l'une quelconque des revendications 1 ou 6, caractérisé en ce que le corps fileté (2) présente à son extrémité opposée à la partie de réception (3) une section (13) d'un premier diamètre et une section (14) limitrophe de celle-ci, d'un second diamètre plus grand que le premier diamètre.

11. Dispositif de positionnent selon l'une quelconque des revendications 1 ou 6, caractérisé en ce que le plan de la fente (4) s'étend de manière sensiblement parallèle à l'axe longitudinal de la partie formant corps fileté (2).

12. Dispositif de positionnement selon l'une quelconque des revendications 1 ou 6, caractérisé en ce que les faces extérieures du fond de la fente (4) sont abaissées de façon à permettre un pivotement de la tige (5) autour du centre (12) du segment de sphère ou autour de l'axe du segment de cylindre.
